# EUROPEAN PATENT SPECIFICATION

(11) **EP 1 534 186 B1**
(45) Date of publication and mention of the grant of the patent: **04.07.2007**
(21) Application number: 02793116.1
(22) Date of filing: 27.12.2002
(51) Int. Cl.: A61F 2/08, A61B 17/04

(54) **ANCHOR DEVICE FOR FIXING A SINEW**
VERANKERUNGSVORRICHTUNG ZUR FIXATION EINER SEHNE
DISPOSITIF D'ANCRAGE DESTINE A FIXER UN TENDON

(30) Priority: 31.12.2001 DE 20121088 U
(43) Date of publication of application: 01.06.2005
(73) Proprietor: Pakdaman, Saeed, 81245 München (DE)
(72) Inventor: Pakdaman, Saeed, 81245 München (DE)
(86) International application number: PCT/EP2002/014767
(87) International publication number: WO 2003/055415

(56) References cited:
- FR-A- 2 769 206
- US-A- 5 354 298
- US-A- 6 146 408
- US-B1- 6 270 518
- US-B1- 6 306 158

## Description

The invention relates to a device for an endoostale fixture of a sinew (x) in the joint area according to the introducing part of the main claim.

In US -6 270 518 an anchor device for fixing a sinew in the area of a joint whereas a longish, to the ends reduced curved shaped part with a longside deepening and a hole through the transverse axis is performed, wherein the shaped part has a profile.

Known are surgery processes using suture and buttons in order to fix loosened sinews or transplantates. Because of the low tensile strength of these methods several complicated mechanical fixing devices were developed.

It is the object of this invention to simplify existing fixing methods and to reduce the number of implantant parts.

Compared to the existing devices this device is compact and easy to handle.

Because of the special performance of this device, a quick and simple but longterm fixation in a drilling hole of a bone is possible. This further enables an atraumatic and simple surgery in the drilling hole of the bone.

This kind of fixing leads to an optimum hold of a transplantat of, for example ligaments or sinews and withstands the tensile strengths caused by normal body stresses.

This device and it's implantation does not affect or hurt the lateral cortex of the femur and the muscles of the respective side, which also prevents atrophy and destruction of cells.

The method reduces possible damages, because of the low amount of material not being human body related.

This device therefore enables an uncomplicated , quick implantation, which once positioned, is then fixed tightly at it's place.

The special advantage of this device consists in inserting a preselected sinew, which can be for instance the Semitendinosus-sinew or other therefore applied ligaments, - also synthetically produced- , in the femur.

Therefeore a special use of this device is the fixing of one of the front universal ligaments in the proximal area.

But also for different types of joints, like for instance for hip joints or ankle joints, this device can be used.

Another advantage of the device is the fixing of the transplantat close to the joint. This avoids extended strain stress of the sinew, the so calles bungee-effect.

The mass, as well as the size of this device are variables which means that the respective use, depending on the type of joint and size and diameter of the sinew determine these variables.

Taking the use of this device for a Semitendinosus-sinew as an example, the following design parameters are characteristic:

The diameter of hole (1) of the device is 5mm=a, which enables to thread the

Semitendiosus-sinew.

The respective diameter of the device is b= 10mm and the height is c=15mm. The radius of the turning edges (7), which means the upper and lower end of the device is d= 2,5mm. This leads to a characteristic design relation of b=2a; c=3a; d=0,5a

Using more solid materials like Titan, or in case of thinner sinews, the size relation of the device structure will change. The hole (1) or the total size of the device for instance could be smaller or larger.

In general, the small size of the implantat does not extend the reconstruction time, which means the growing of the bone material around the implantat.

In the following the invention will be decribed in more detail, using the figures:
- Fig. 1a-c: Different sights of the anchor device
- Fig. 2: Device with transplantat
- Fig. 3: Device with inserting- and fixing thread
- Fig. 4: Device in the drilling hole of a bone with transplantat and threads

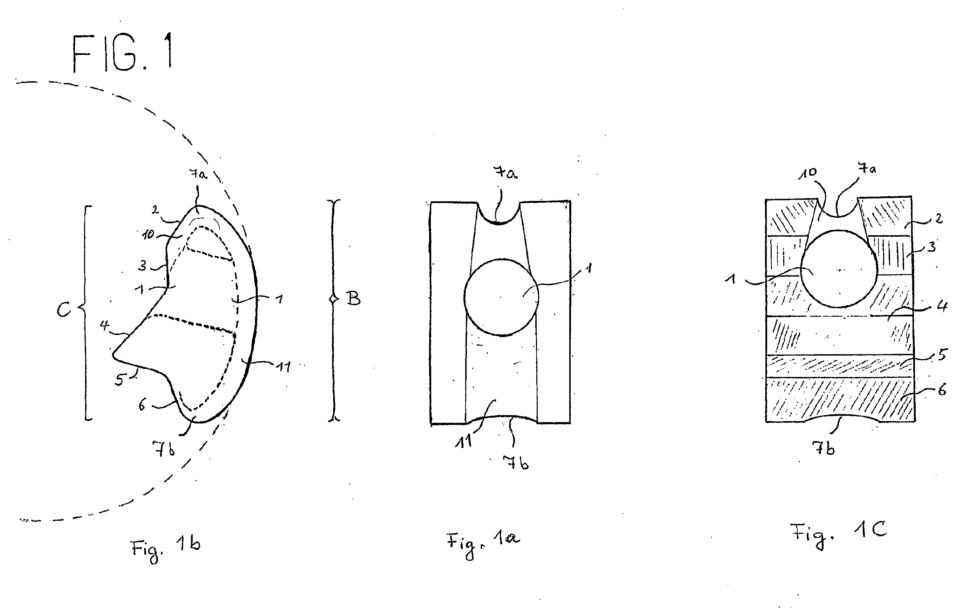

In the following the invention will be described in more detail using figures 1 a to 1 c.

The device is performed as a shaped part preferably made out of metal or plastics or bone material.

The invention is based on an appropriate shaped part which can be inserted in a drilling hole.

This device has a longitudinal to the ends reduced and curved design with a deepening surrounding fully or partially two of the longitudinal sides of the shaped part. In addition a transversal arranged hole connects the opposite sides of the deepening.

The device consists of four longitudinal sides. Two longitudinal sides of the shaped part are formed, one has an arch-formed-, the second has a step-formed profile. Both sides are placed in opposite. Two longitudinal sides are flat and are also placed in opposite,

Fig. 1a shows the arch-formed area with one deepening 11 wich reaches from the so called distal position , which means lower end 7b, to the so called proximal position, which means the upper end 7a of the device. The transversal hole 1 is for example placed close above the center.

The proximal edge 7a of the device is the insertion direction of the device through the drilling hole 13 of the bone.

Fig. 1b shows the profil of the device with the arch-formed area B and a step-formed area C, consisting of the partial areas 2, 3, 4, 5 and 6 which are arranged stepwise displaced to each other.

Fig. 1c shows the step-formed area of the device, consisting of the flattened partial areas 2, 3, 4, 5 and 6, whereas area 4 is connected with area 2 by a step.

From hole 1a deepening 10 reaches to the proximal end 7a and is connected with the deepening 11 on the opposite side.

The area below the hole in Fig.1c has a curved shaped deepening. The distal end of the edge of this side has a curved shaped deepening.

All edges of the device are smoothly curved. This enables an atraumatic and simple insertion in the drilling hole of the bone. In addition, this arrangement results in more stability and reduces the damage of the edges and the sinew in case of tensile stress on the device.

The outer edges of the hole 1 , as well as the turning edge 7 of the proximal end are curved in that way, that an injury of the sinew or a transplantat is prevented.

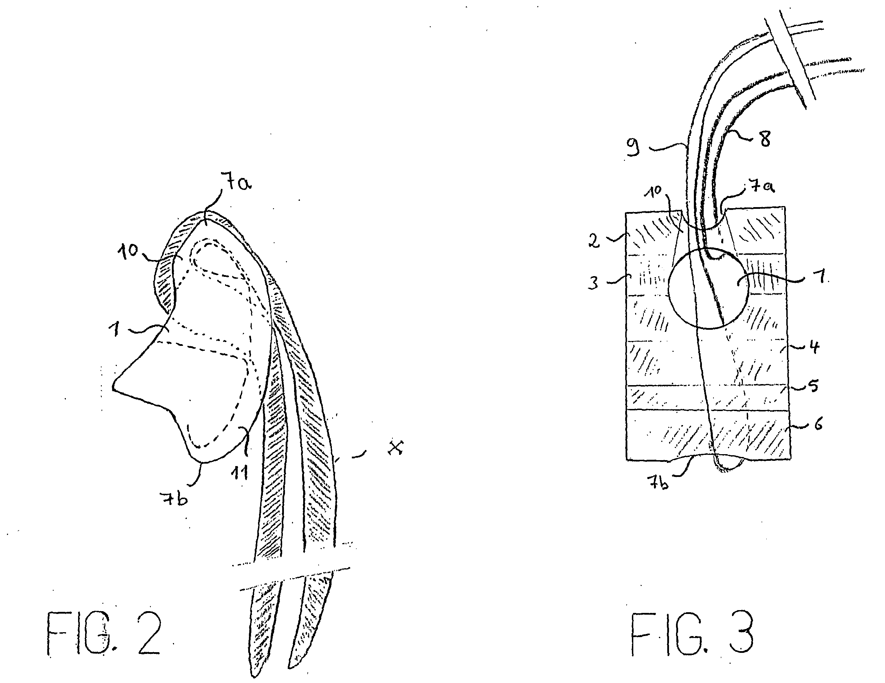

Fig. 2 shows the device and the position of the transplantat inside the device.

One end of the transplantat is threaded from the distal end 7b through the deepening 11 into hole 1 and is led through deepening 10 via the proximal edge 7a back to deepening 11, in such a way that both ends of the transplantat can be put together on deepening 11.

The device consists of appropriate materials, especially biological absorbing materials, e.g. Titan or bone material. Also appropriate plastic materials can be used.

Fig. 3 shows the device with insertion and fixing thread.

The thread 8 is pulled through hole 1 in opposite direction to the transplantat x and is placed as a loop in deepening 10/11 facing the proximal end 7a.

A second thread 9 is put over the proximal edge 7a through deepening 10 and hole 1, further through deepening 11 and via the distal edge 7b, then in the deepening of the area 4 and back in direction of the proximal edge 7a.

Thread 9 is not obligatory.

Thread 8 is used later to pull the device in the drilling hole of the bone 13 and with the help of thread 9 the device including the connected transplantates can be positioned and fixed -by falling over and tightening of the device- inside the prepared drilling hole 13 in the bone.

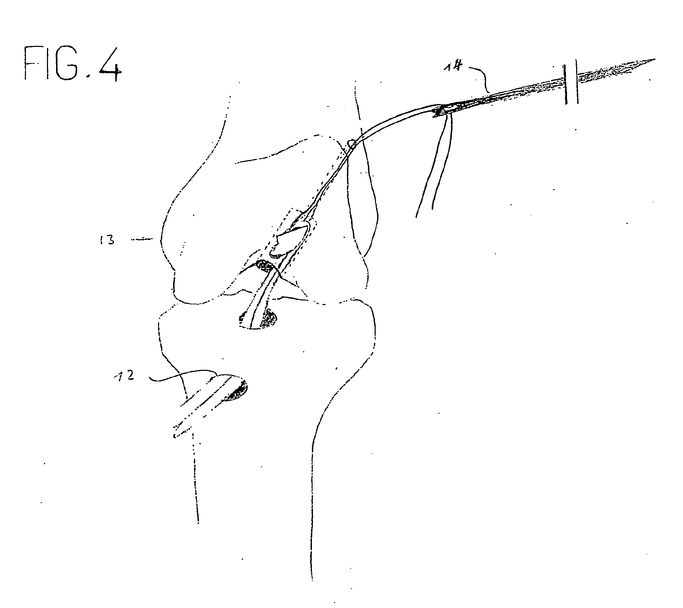

Fig. 4 shows the placement of the device inside the drilling hole of a bone.

The device including the Semitendinosus-sinew x is pulled through a transition hole 12 in the drilling hole 13 in the Fossa Intercondylaris on the femur side of the knee-joint. Before the two threads 8/9 were connected in an eye of a wire or needle 14.

The profile of the device enables a simple insertion in the drilling hole 13 of the bone. Inside the channel of the drilling hole 13 the device can be moved and positioned by a tensile force of the transplantat x which results in a falling over of the device inside the drilling channel.

If necessary, the falling over of the device can be forced with the help of thread 9, if not possible with the transplantat.

The device can move around it's axis because of the lonitudinal arch-formed side and is fixed inside the drilling hole after the falling over.

Because of the step formed profile of the opposite side the device is tensioned mechanically at the inner wall of the bone. As a result, a further movement of the device is prevented and the device is fixed until the reconstruction of the bone with rebuildt bone material tightly surrounding the device has finished.

After the mounting process the distal ends of the sinew are fixed at the Tibia.

## Claims

1. Anchor device for fixing a sinew in the area of a joint comprising a longish, to the ends reduced curved shaped part having distal and proximal ends (7b,7a) wherein the shaped part has a profile with one longitudinal side having an arched-formed structure (B) and an opposite longitudinal side having a step-formed structure (C), **characterized in that** the device comprises longitudinal deepenings (10,11) extending along each of said longitudinal sides and connected with each other at the proximal end and by means of a transversally arranged hole (1) for receiving said sinew.

2. Device according to claim 1, **characterised in that** the longitudinal deepenings are fully surrounding the arch-formed longitudinal side and partly surrounding the step-formed longitudinal side.

3. Device according to claim 1 to 2, **characterised in that** the deepening formed at the arch-formed longitudinal side of the shaped part connects the deepening of the opposite longitudinal side at one end of the shaped part.

4. Device according to claim 1 to 2, **characterised in that** the hole connects the longitudinal sides with the arch-formed and the step-formed profile and the deepenings of these sides to guide the sinew or thread

5. Device according to claim 1, **characterised in that** the step-formed side has a wedge or nose formed profile.

6. Device according to claim 1 to 3, **characterised in that** the deepenings are **characterized by** a curved shaped form.

7. Device according to any of the claims 1 to 6, **characterised in** tapered or flattened ends.

8. Device according to any of the claims 1 to 7, **characterised in that** the shaped part consists of biological absorbing materials or metals like Titan or plastics or one's own or others bone material.

9. Device according to any of the claims 1 to 8 charaterised in consisting of one piece.

10. Device according to any of the claims 1 to 9 **characterised in that** the shaped part has a cylindric or squared basic form.

## Patentansprüche

1. Ankervorrichtung zur Fixierung einer Sehne im Gelenkbereich, **dadurch gekennzeichnet, dass** es ein längliches, sich zu den Enden verjüngendes abgerundetes Formteil mit einer längslaufenden Vertiefung und einer quer durch das Formteil verlaufender Bohrung ist, bei der das Formteil im Profil derart ausgebildet ist, dass eine Längsseite bogenförmig ist und die gegenüberliegende Längsseite eine stufenförmig verlaufende Kontur mit einer oberhalb der Bohrung längslaufenden Vertiefung aufweist.

2. Vorrichtung nach Anspruch 1, bei der die stufenförmige Seite durch einen hervorstehenden keil- oder nasenförmigen Absatz **gekennzeichnet** ist.

3. Vorrichtung nach Anspruch 1 bis 2, bei der die Vertiefungen rundförmig ausgebildet sind.

4. Vorrichtung nach einem der Ansprüche 1 bis 3, bei der die längslaufende Vertiefung ganz oder teilweise umlaufend ist.

5. Vorrichtung nach einem der Ansprüche 1 bis 3, bei der die Enden des Formteils spitz oder abgeflacht sind.

6. Vorrichtung nach einem der Ansprüche 1 bis 3, bei der die Bohrung (1) beide gegenüberliegende Vertiefungen verbindet.

7. Vorrichtung nach einem der Ansprüche 1 bis 6, bei der alle Kanten des Formteils abgerundet sind.

8. Vorrichtung nach einem der Ansprüche 1 bis 7, bei der das Formteil aus bioabsorbierbaren Materialen oder Metall, insbesondere Titan, oder Kunststoff oder eigenem oder fremden Knochen besteht.

9. Vorrichtung nach einem der Ansprüche 1 bis 8, bei der das Formteil nur aus einem Stück besteht.

10. Vorrichtung nach einem der Ansprüche 1 bis 9, bei der das Formteil eine zylindrische oder quaderförmige Grundform hat.

## Revendications

1. Dispositif d'ancrage destiné à la fixation d'un tendon dans la zone ce qu'il est constitué d'une pièce préformée oblongue arrondie allant en se rétrécissant vers les extrémités et dotée d'un évidement s'étendant dans le sens longitudinal et d'un alésage traversant transversalement la pièce préformée, dans le cadre duquel la pièce préformée est conçue de profil de sorte qu'un côté longitudinal est en forme d'arc et qu'un côté longitudinal opposé présente un contour à paliers avec un évidement s'étendant longitudinalement au-dessus de l'alésage.

2. Dispositif selon la revendication 1, dans le cadre duquel le côté à paliers est **caractérisé par** un épaulement cunéiforme ou en forme de nez faisant saillie.

3. Dispositif selon les revendications 1 à 2, dans le cadre duquel les évidements sont de forme ronde.

4. Dispositif selon l'une des revendications 1 à 3, dans le cadre duquel l'évidement s'étendant dans le sens longitudinal fait partiellement ou complètement le tour de la pièce préformée.

5. Dispositif selon l'une des revendications 1 à 3, dans le cadre duquel les extrémités de la pièce préformée sont effilées ou aplaties.

6. Dispositif selon l'une des revendications 1 à 3, dans le cadre duquel l'alésage (1) relie les deux évidements opposés.

7. Dispositif selon l'une des revendications 1 à 6, dans le cadre duquel toutes les arêtes de la pièce préformée sont arrondies.

8. Dispositif selon l'une des revendications 1 à 7, dans le cadre duquel la pièce préformée est composée par des matériaux ou des métaux biologiquement absorbables, notamment par du titane, ou par une matière synthétique ou par de l'os prélevé sur le patient ou de l'os prélevé sur un autre patient.

9. Dispositif selon l'une des revendications 1 à 8, dans le cadre duquel la pièce préformée n'est réalisée que d'une seule pièce.

10. Dispositif selon l'une des revendications 1 à 9, dans le cadre duquel la pièce préformée présente une forme de base cylindrique ou carrée.
